# EUROPEAN PATENT SPECIFICATION

(11) **EP 0 668 349 B1**
(45) Date of publication and mention of the grant of the patent: **13.11.2002**
(21) Application number: 95830039.4
(22) Date of filing: 15.02.1995
(51) Int. Cl.: C12N 9/22, A61K 38/46, A61K 47/48, G01N 33/563

(54) **Muteins of pancreatic ribonuclease and uses thereof**
Pankreatische ribonuclease Mutante und ihre Verwendung
Mutants de ribonucléase pancréatique et leur utilisation

(30) Priority: 16.02.1994 IT RM940073
(43) Date of publication of application: 23.08.1995
(73) Proprietor: UNIVERSITA' DEGLI STUDI DI NAPOLI, I-80138 Napoli (IT)
(72) Inventor: D'Alessio, Guiseppe, Università degli Studi di, I-80134 Napoli (IT); Di Donato, Alberto, Università degli Studi di, I-80134 Napoli (IT); Piccoli, Renata, Università degli Studi di, I-80134 Napoli (IT)
(74) Representative: Taliercio, Antonio

(56) References cited:
- US-A- 5 389 537
- J.BIOL.CHEM, vol. 253, no. 7, 10 April 1978, pages 2070-2072, XP002007716 WILSON : "EFFECT OF REDUCTIVE LACTOSAMINATION ON THE HEPATIC UPTAKE OF BOVINE PANCREATIC RIBONUCLEASE A DIMER"
- J.BIOL.CHEM, vol. 266, no. 31, 5 November 1991, pages 21202-21207, XP002007717 RYBAK ET AL: "CYTOTOXIC POTENTIAL OF RIBONUCLEASE AND RIBONUCLEASE HYBRID PROTEINS"
- J.BIOL.CHEM., vol. 269, no. 26, 1 July 1994, pages 17394-17396, XP002007718 DI DONATO ET AL: "RIBONUCLEASE A CAN BE TRANSFORMED INTO A DIMERIC RIBONUCLEASE WITH ANTITUMOR ACTIVITY"
- FEBS LETTERS, vol. 359, 6 February 1995, pages 31-34, XP002007719 CAFARO ET AL: "THE ANTITUMOR ACTION OF SEMINAL RIBONUCLEASE AND ITS QUATERNARY CONFORMATIONS"
- MOL. BIOL. EVOL. vol. 7, no. 1, January 1990, pages 29-44, SCHULLER ET AL:'EVOLUTION OF NUCLEIC ACIDS CODING FOR RIBONUCLEASES: THE mRNA SEQUENCE OF THE MOUSE PANCREATIC RIBONUCLEASE'

## Description

The invention relates to stable dimeric muteins of pancreatic ribonuclease (RNase), having an antitumoral activity, to be used to develop pharmaceutic compositions.

The bovine pancreatic ribonuclease A (RNase A) belongs to the RNase family. The sequence and the biologic and chemical features of this enzyme are described in the reference (1) and (2), herein incorporated. RNase A, that is a very well investigated protein, is a monomeric protein, as all proteins belonging to this family, except the bovine seminal ribonuclease (BS RNase) (3), which is a homodimeric protein. However, BS-RNase subunits show a sequence identity with RNase A higher than 80%. Seminal RNase is a ribonuclease having peculiar biologic activities (4), including a very effective antitumoral activity (5,6).

RNase A dimers were obtained by Crestfield, Stein and Moore (7) through a freeze-drying process from acetic acid; these dimers were poorly stable and appeared to be derived from a N-terminal exchange (8), that also occurs in the seminal dimeric RNase (9). Also it was demonstrated that the dimerization did not lead to only one quaternary structure; actually, two mutually balanced protein forms could exist, a first form, wherein the subunit interchange occurs (MxM form), and another form (M=M), wherein the subunit interchange does not occur (10). A balance is obtained when the ratio MxM:M=M is 2:1.

Bartholeyins and Moore developed RNase A stable dimeric derivatives through bonding the monomers with two-function reagents (11). These dimeric compositions consisted of mixtures of various dimeric derivatives, which were generated because of random bonds among lysine different residues and showed an antitumoral action, though being less effective than the seminal enzyme activity (12).

The authors had previously demonstrated that chimeric proteic molecules, obtained through DNA manipulations and consisting of the 1 to 40 BS-RNase aminoacid residues, as well as of the 41 to 124 aminoacid of the bovine RNase A, aggregated to form stable active dimers (14). However, these chimeric molecules showed no anti-tumoral activity.

The authors now identified the amino acid residues, as well as their position in the pancreatic RNase sequence, that, after having been properly mutated, produced muteins, that are able to dimerize spontaneously to form chemically defined and stable dimeric compounds. Such pancreatic RNase derivatives could be advantageously used, as having provided an anti-tumoral activity.

Among the practicable substitutions on various aminoacid residues, the authors selected four minimal residues, making the mutein dimeric and showing an effective level of antitumoral activity. Furthermore the muteins showed to be stable, chemically defined and able to be produced in a large extent in a controlled system, such as E. coli, with no risk of contaminations, as occurred for recombinant proteins from superior organisms. Moreover, these molecules could be submitted further to chemical derivation processes.

Accordingly, it is an object of the invention a pancreatic RNase dimeric mutein, having an antitumoral activity characterised in that the mutations consist of the amino acid residues as follows in the indicated positions: pro 19; leu 28; cys 31 and cys 32. Pancreatic RNase, as used herein, means Rnase from mammalian organisms as bovine, human, ovine, murine or rabbit pancreas.

Moreover, it is another object of the invention a pharmaceutic composition, having an antitumoral activity, comprising the mutein of this invention in a pharmaceutically acceptable amount.

It is also an object of the invention a conjugate anti-tumoral cell antibody-mutein to develop immunotoxins, having antitumoral activity, to be specifically and selectively targeted to the site of action. Such immunotoxins could be obtained through chemical procedures, by binding the immunoglobulin to the mutein of the invention, using bifunctional reagents and methods that are well known to the skilled in the art., e.g., as showed by Newton et al. (14) for an anti-transferrin antibody. As an alternative, immunotoxins could be obtained through forming disulfide bridges between the mutein monomeric subunit and a FAB fragment of monoclonal antibodies, directed against specific epitopes of tumoral cells, both comprising SH groups.

The invention will be now described as related to some preferred embodiments, by reference to the following figures, wherein:
Fig.1 is a polyacrylamide gel (15%, SDS-PAGE) electrophoresis under non reducing conditions (columns 1, 2 and 4), and under reducing conditions (column 3). Column 1: RNase A, sigma type XIIA; Column 2: BS-RNase, purified from seminal vesicles, according to (20); Columns 3 and 4: PLCC RNase A;
Fig.2 is a filtered gel chromatography on a G75 Sephadex™ column (0.9 x 47.5) of PCCL RNase after incubation (24 µM) in 0.1 M of Tris HCl, pH 8.4, with DTT (240 µM) for 30 minutes. A: PLCC RNase; B: PLCC RNase after incubation for 60 hours at 37°C at pH 7.0; and

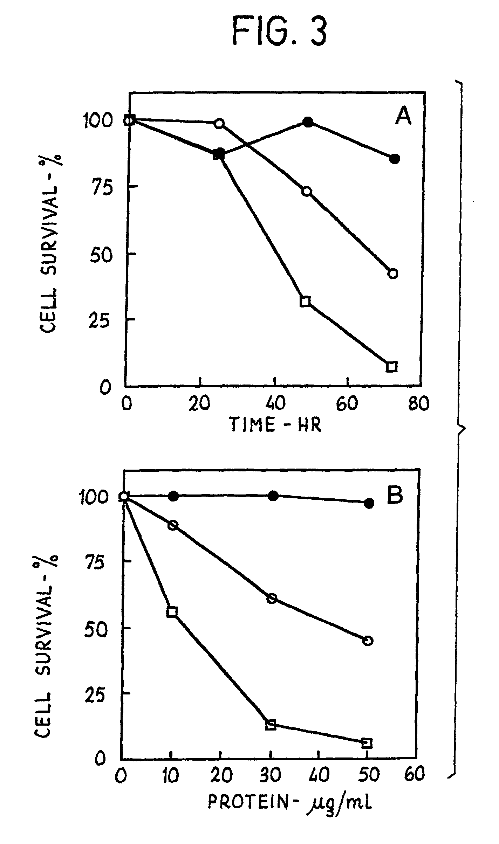

Fig.3 shows fibroblast (ATCC, Richmond, U.S.A) survival diagrams, that were cultivated in presence of PCCL RNase A ( 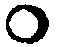 ); RNase (●) and BS-RNase ( 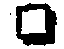 ). A: survival of cells grown in presence of 30 µM of each RNase; B: dose-response curve after 72 hour growth in presence of each RNase. Values were calculated as compared with control cells, that were cultivated in absence of Rnase, taken as 100%. Cells were plated on 24 well trays in a DMEM culture medium, comprising glutamine, penicillin, streptomycin and 10% of FCS, at a concentration of 5 x 10⁴ cells/ml. RNase was added to the culture media before the cell plating to the trays.

The cDNA for RNase A was obtained, as described in (15) and mutated by means of a PCR mutagenesis at 5' and 3' terminals to allow to be inserted into Eco RI and Sal I sites of the pUC118 plasmid. Then, the plasmid was mutagenized according to the method of Kunkel et al. (16). The mutated cDNA was expressed in E. coli, as already indicated as for cDNA for the seminal RNase (17). In other words, DNA was inserted into the pT7-7 expression vector, as described in (16), after a PCR mediated, further 5'end mutagenesis to allow the polar insertion into the vector at Nde I and Sal I sites. The resulting plasmid was expressed in the JM109 (DE3)pLysS E. coli strain.

The mutated protein, as included into inclusion bodies, was denaturated, purified and renatured as a catalitically active protein, in presence of an oxyde reducing buffer with glutatione, as described for the seminal RNase, that was expressed in the same expression system (15). The purified protein was homogenous, as shown through an analysis, using SDS (Page), having an identical specific activity against the yeast RNA as shown by the native pancreatic RNase (100 ± 9 K units per mg). A sequence analysis, using a 475 A sequencer (Applied Biosystem) of the first 35 residues of the recombinant protein showed that the N terminal residue was methionine, followed by the first 34 RNase A residues, except the 19, 28, 31 and 32 residues, which were Pro, Leu, Cys and Cys respectively.

From a protein sample, subjected to an electrospray type mass spettroscopy (15), multicharged ions were obtained, from which the protein average molecular mass was calculated to be appr. 14.425 ± 2.35 Da, i.e, exactly corresponding to the expected figure.

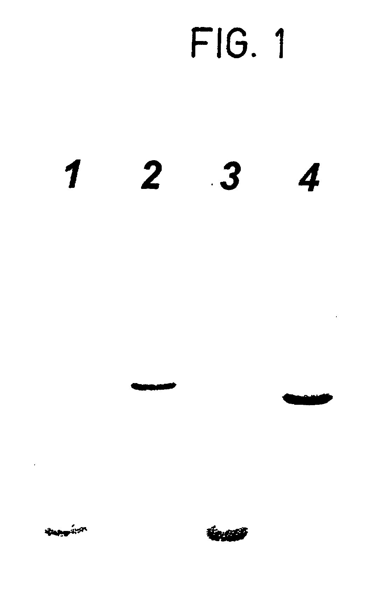

When the glutatione protecting components were removed fron the RNase mutein through a reduction procedure under not severe conditions (15), free monomers formed easily dimers. The RNase A dimeric structure, as well as the existing disulfide bridges, were identified through an analysis, using SDS (PAGE), as shown in fig.1. In non reducing conditions, RNase migrated as did the seminal RNase, while, under reducing conditions, migrated as did the natural A RNase.

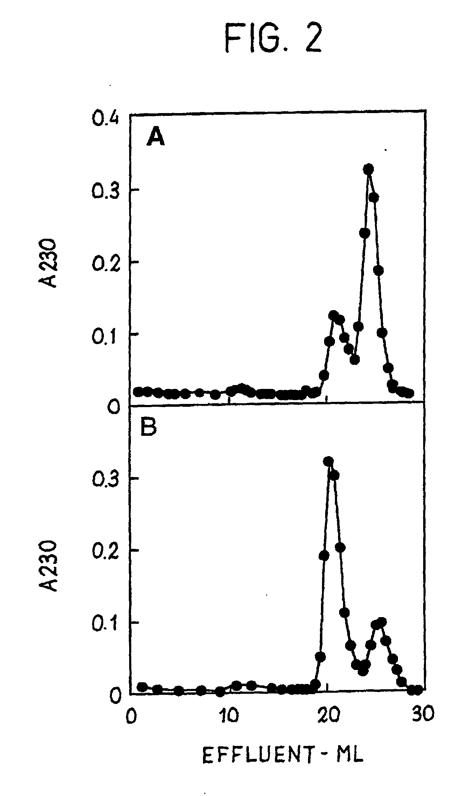

In order to detect whether the MxM dimeric form was present, as in the cases of the seminal RNase dimers and of the RNase A when aggregated from acetic acid solutions, the sulfide bridges in a PLCC RNase mutein sample were selectively cut and an analysis of the obtained materials carried out through a gel filtering process. When submitted to a process as above, the (non exchanging) M=M dimers dissociated as monomers because of the disulfide bridge removal, and eluted as monomers from the gel filtering column; a detection of a dimeric form pattern (not covalent dimers) on the gel filtering column showed that MxM dimers existed, as the N terminal segment exchange prevented the dissociation (10). Fig.2, A shows that appr. a 25% of the selectively reduced RNase mutein separated as not covalent dimers, while the remaining fraction eluted as free monomers. This finding, as confirmed by a number of indipendent tests, demonstrated that appr. one fourth of the RNase mutein was in MxM form.

The tests were repeated using a RNase mutein that was incubated for 60 hours at 37°C at pH 7, i.e., under such conditions, that enable the seminal RNase M=M form to convert to the MxM form and to balance both forms (10). The fig. 2 B shows that the not covalent dimers were appr. two thirds of the not reduced protein, after having been incubated under the conditions as above. This means that also the RNase A mutein, as the seminal RNase A, existed in two dimeric forms and, under balanced conditions, the MxM/M=M ratio is 2:1.

When SVT2 fibroblasts (BalbC 3T3 cells transformed with the SV40 virus) were grown in presence of 30 µg/ml of RNase A mutein, an inhibition of the cell growth was observed (fig. 3 A). While the wild RNase A did show no effect, the RNase dimeric mutein, after having been incubated for 72 hours, showed to inhibit the cell growth more than the 50%. A dosing curve, as shown in fig. 3 B, confirmed that the cytotoxic effect of the RNase A mutein against the malignant cells was selective, as well as the wild RNase A was not effective. Furthermore, through isolating the mutein MxM form, as described in (10), only this form was identified as inhibiting the tumoral cells to replicate. The RNase cytotoxic action was selective against tumoral cells, as the protein was not effective at all against the non malignant parental line of 3T3 fibroblasts. Moreover, by separating the M=M and MxM forms, as described for the BS-RNase in (10), only the MxM form showed an antitumoral activity.

As shown in fig. 3, the RNase A mutein inhibiting effect on the converted fibroblast growth is less strong than the seminal RNase effect. Probably, while the dimeric structure is an essential prerequisite for the antitumoral RNase, this structure could be not sufficient to exploit the protein whole cytotoxic activity, as other proteic factors could be involved.

### REFERENCES

1. Richards, F.M. and Wyckoff, H.W.: "The Enzymes" (3rd ed.) 4, 647 (1971)
2.Blackburn, P. and Moore, S.: "The Enzymes" (3rd ed.) 15, 417 (1982)
3. D'Alessio, G., Di Donato, A., Parente, A. and Piccoli, R.: "Trends Biochem. Sci." 16, 104 (1991)
4. D'Alessio, G.: "Trends Cell Biol." 3, 106 (1993)
5. Vescia, S., Tramontano, D., Augusti-Tocco, G. and D'Alessio G.: "Cancer Res." 40, 3740 (1980)
6.Laccetti, P., Portella, G., Mastronicola M.R., Russo, A., Piccoli, R. and D'Alessio, G.:"Cancer Res." 52, 4582 (1992)
7.Crestfield, A.M., Stein, W.H.and Moore, S.: "Arch. Biochem. Biophys. Suppl." 1, 217 (1962)
8.Crestfield, A.M. and Fruchter, R.G.: "J. Biol. Chem." 242, 3279 (1967)
9.Capasso, S., Giordano, F., Mattia, C.A., Mazzarella, L. and Zagari, A.:"Biopolymers" 22, 327 (1983)
10.Piccoli, R., Tamburrini, M., Piccialli, G., Di Donato, A., Parente, G. and D'Alessio, G.:"Proc. Natl.Acad. Sci. USA" 89, 1870 (1992)
11.Bartholeyns, J. and Moore, S.: "Science" 186, 444 (1974)
12.Bartholeyns, J. and Baudhuin, P.: "Proc. Natl. Acad. Sci. USA", 73, 573 (1976)
13.Di Donato, A., Cafaro, V., de Nigris, M., Rizzo, M. and D'Alessio, G.: "Biochem. Byophys. Res. Comm." 194, 1440 (1993)
14.Newton, D. et al.: "J. Biol. Chem." 266, 21202-7 (1991)
15.de Nigris, M., Russo, N., Piccoli, R., D'Alessio, G. and Di Donato, A.: "Biochem. Biophys. Res. Comm.", 193, 155 (1993)
16.Kunkel et al.: "Meth. Enzymol." 154, 367 (1987)
17.Studier, F.W. et al.: "Meth. Enzymol." 185, 60 (1990)

## Claims

1. A dimeric mutein of pancreatic RNase having an antitumoral activity **characterised in that** the mutations consist of the amino acid residues as follows in the indicated positions: pro 19; leu 28; cys 31 and cys 32.

2. A pharmaceutic composition having an antitumoral activity, comprising the mutein according td claim 1 in a pharmacologically acceptable amount.

3. A conjugate anti-tumoral cell antibody-mutein according to claim 1, to develop immunotoxins having an antitumoral activity.

## Patentansprüche

1. Dimere Mutein der pankreatischen Rnase, wobei die Mutein eine anti-tumorale Aktivität hat, **dadurch gekennzeichnet, dass** die Mutationen besteht aus folgenden Rückständen in den verzeichneten Positionen: PRO 19, LEU 28, CYS 31 und CYS 32.

2. Pharmazeutische Zusammensetzung, die eine anti-tumorale Aktivität hat, wobei die Mutein nach Anspruch 1 in einer pharmazeutisch annehmbaren Menge enthalten wird.

3. Konjugierte Antikörper - Anti-Tumorzelle - Mutein nach Anspruch 1, für die Entwicklung von Immuno-Toxinen, die eine anti-tumorale Aktivität haben.

## Revendications

1. Mutéine dymérique de la Rnase pancréatique, ayant une activité anti-tumorale, **caractérisée en ce que** les mutations consistent en résidus aminoacides, dans les positions indiquées comme suit : pro 19, leu 28, cys 31 et cys 32.

2. Composition pharmaceutique ayant une activité anti-tumorale, comprenant la mutéine selon la revendication 1 en une quantité pharmacologiquement acceptable.

3. Conjugué anticorps - anti-cellules tumorales - mutéine selon la revendication 1, pour le développement d'immuno-toxines ayant une activité anti-tumorale.
